# EUROPEAN PATENT APPLICATION

(11) **EP 1 714 654 A1**
(43) Date of publication of application: **25.10.2006**
(21) Application number: 05709638.0
(22) Date of filing: 02.02.2005
(51) Int. Cl.: A61K 31/501, A61P 9/00, A61P 9/10, C07D 401/12

(54) **DRUG FOR INHIBITING VASCULAR INTIMAL HYPERPLASIA**

(30) Priority: 09.02.2004 JP 2004032551
(71) Applicant: Nissan Chemical Industries, Ltd., Chiyoda-ku, Tokyo 101-0054 (JP); TAISHO PHARMACEUTICAL CO., LTD, Tokyo 170-8633 (JP)
(72) Inventor: NISHIYAMA, Hiroshi, c/o Nissan Chemical Ind. Ltd., Minamisaitama-gun, Saitama 3490218 (JP); SHUDO, Norimasa, c/o Nissan Chemical Ind., Ltd., Minamisaitama-gun, Saitama 3490218 (JP); TSURUZOE, Nobutomo, c/o Nissan Chemical Ind. Ltd., Minamisaitama, Saitama 3490218 (JP)
(74) Representative: Hartz, Nikolai
(86) International application number: PCT/JP2005/001518
(87) International publication number: WO 2005/074938

(57) **Abstract**

To provide an intimal hyperplasia inhibitor useful for prevention of restenosis after percutaneous transluminal coronary angioplasty (PTCA) or vascular stent placement or treatment of its progress.

An intimal hyperplasia inhibitor containing a 3(2H)-pyridazinone compound represented by the formula (I): 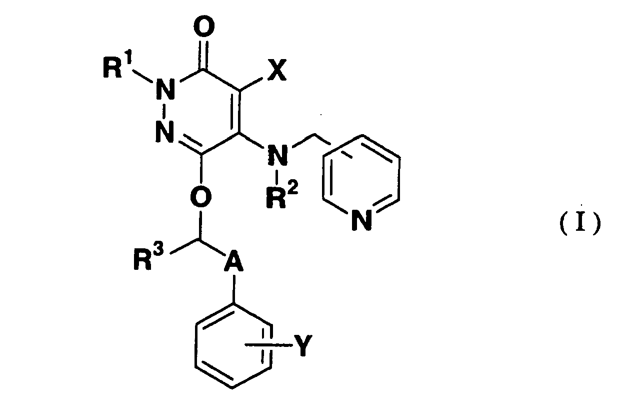 [wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group] or a pharmacologically acceptable salt thereof.

## Description

### TECHNICAL FIELD

The present invention relates to a vascular intimal hyperplasia inhibitor containing a pyridazinone compound or a pharmacologically acceptable salt thereof as an active ingredient.

### BACKGROUND ART

In the pathogenic mechanism of myocardial infarction and angina pectoris, coronary stenosis due to arterioscloerotic intimal hyperplasia is considered as a major cause. On the other hand, in recent years, percutaneous transluminal coronary angioplasty (PTCA) has been performed widely to dilate stenotic lesions of the coronary artery with intimal hyperplasia, and vascular stent placement is performed increasingly. However, it is a major medical problem that vascular endothelial cells exfoliate after PTCA or vascular stent placement, and reocclusion occurs due to intimal hyperplasia accompanied by proliferation of vascular smooth muscle cells. Therefore, highly safe drugs which selectively inhibit vascular intimal hyperplasia are promising as drugs useful not only for prevention and treatment of arteriosclerotic diseases but also for prevention of restenosis after PTCA or vascular stent placement.

Pyridazinone compounds or their salts are known to have excellent antiplatelet action, cardiotonic action, vasodilator action, anti-SRS-A (Slow Reacting Substance of Anaphylaxis) action, thromboxane A2 synthetase inhibitory action, therapeutic action on spinal canal stenosis and erectile dysfunction and angiogenesis stimulatory and enhancing action and are promising as antiplatelet agents (Patent Documents 1 to 6).

However, no reports have been made on what effect these pyridazinone compounds have on vascular intimal hyperplasia. On the other hand, though among various treatments for vascular intimal hyperplasia, pharmacotherapy is an established one, a better pharmacotherapy is demanded.
Patent Document 1: JP-B-7-107055
Patent Document 2: JP-A-7-252237
Patent Document 3: JP-A-7-285869
Patent Document 4: WO99/11268
Patent Document 5: WO00/12091
Patent Document 6: WO00/33845

### DISCLOSURE OF THE INVENTION

### PROBLEMS THAT THE INVENTION IS TO SOLVE

The object of the present invention is to provide an excellent vascular intimal hyperplasia inhibitor.

### MEANS OF SOLVING THE PROBLEMS

As a result of their extensive research, the present inventors have found that the pyridazinone compounds represented by the following formula (I) or their pharmacologically acceptable salts have excellent inhibitory action on vascular intimal hyperplasia and have accomplished the present invention.

Namely, the present invention provides a vascular intimal hyperplasia inhibitor containing a 3(2H)-pyridazinone compound represented by the formula (I) or a pharmacologically acceptable salt thereof as an active ingredient. 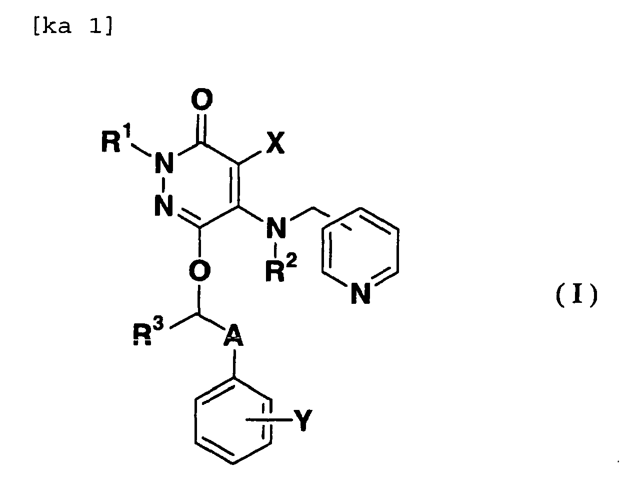 [wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group].

The vascular intimal hyperplasia inhibitor of the present invention is preferably a pyridazinone compound of the formula (I) wherein R¹ and R² are hydrogen atoms, R³ is a hydrogen atom or a C₁₋₄ alkyl group, X is a halogen atom, Y is a halogen atom or a hydrogen atom, and A is a C₁₋₅ alkylene which may be substituted with a hydroxyl group, or a pharmacologically acceptable salt thereof.

The pyridazinone compound represented by the formula (I) in the vascular intimal hyperplasia inhibitor of the present invention is particularly preferably 4-bromo-6-[3-(4-chlorophenyl)propoxy]-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.

### EFFECTS OF THE INVENTION

The present invention provides a novel vascular intimal hyperplasia inhibitor containing a pyridazinone compound (I) or a pharmacologically acceptable salt thereof.

### BRIEF DESCRIPTION OF THE DRAWINGS

[Fig. 1] Fig. 1 shows the intimal area after oral administration of 10 mg/kg of Compound A or 300 mg/kg of Cilostazol in Test Example 1. * indicates that the difference was significant with p < 0.05 as compared with the solvent group by Dunnett's test.
[Fig. 2] Fig. 2 shows the I/M ratio after oral administration of 10 mg/kg of Compound A or 300 mg/kg of Cilostazol in Test Example 1. * indicates that the difference was significant with p < 0.05 as compared with the solvent group by Dunnett's test.

### BEST MODE FOR CARRYING OUT THE INVENTION

Now, the pyridazinone compound represented by the above formula (I) or a pharmacologically acceptable salt thereof in the vascular intimal hyperplasia inhibitor of the present invention will be described.

In the formula (I), the C₁₋₆ alkyl groups as R¹, R² and R³ may be linear or branched and may, for example, be methyl, ethyl, n-propyl, isopropyl, n-butyl, isobutyl, sec-butyl, t-butyl, pentyl, hexyl or the like.

R¹ and R² are preferably hydrogen atoms, and R³ is preferably a hydrogen atom or a C₁₋₄ alkyl group.

The C₁₋₄ alkyl group as R³ may, for example, be methyl, ethyl, n-propyl, isopropyl, n-butyl, isobutyl, sec-butyl, t-butyl or the like. Particularly preferred as R³ is a hydrogen atom.

The halogen atoms as X and Y are fluorine atoms, chlorine atoms, bromine atoms or iodine atoms. X is preferably a halogen atom, and Y is preferably a halogen atom or a hydrogen atom.

The C₁₋₈ alkylene which may be substituted with a hydroxyl group as A may be linear or branched and may, for example, be methylene, ethylene, propylene, butylene, pentylene, hexylene, heptylene, octylene, 2,2-dimethylethylene, 2,2-diethylethylene, 2,2-di-n-propylethylene, hydroxymethylene, 1-hydroxyethylene, 2-hydroxyethylene, 3-hydroxypropylene or the like.

A is preferably a C₁₋₅ alkylene which may be substituted with a hydroxyl group.

In the formula (I), the methylene group may be liked to any position in the pyridine ring with no particular restrictions, but preferably is linked to the 3-position to the nitrogen atom in the pyridine ring.

Further, the substituent Y may be at any position in the benzene ring, but preferably at the 4-position.

Pyridazinone compounds of the formula (I) wherein R¹ and R² are hydrogen atoms, R³ is a hydrogen atom or a C₁₋₄ alkyl, X is a halogen atom, Y is a halogen atom or a hydrogen atom, and A is a C₁₋₅ alkylene which may be substituted with a hydroxyl group are particularly preferred.

As preferable compounds, 4-bromo-6-[3-(4-chlorophenyl)propoxy]-5-(3-pyridylmethylamino)-3(2H)-pyridazinone, 4-bromo-6-[3-(4-chlorophenyl)-3-hydroxypropoxy]-5-(3-pyridylmethylamino)-3(2H)-pyridazinone and their pharmacologically acceptable salts are mentioned.

In the present invention, pharmacologically acceptable salts of the pyridazinone compounds (I) include, for example, salts with inorganic acids (such as hydrochlorides, hydrobromides, phosphates and sulfates), salts with organic acids (such as acetates, succinates, maleates, fumarates, malates and tartrates). These salts may be obtained from the pyridazinone compounds (I) by known methods.

The pyridazinone compounds (I) of the present invention and their pharmacologically acceptable salts cover their stereoisomers and optical isomers. The pyridazinone compounds (I) and their pharmacologically acceptable salts are known compounds and known for their low toxicity. They are obtainable, for example, by the methods disclosed in JP-B-7-107055, USP 5314883, EP-A-482208, JP-A-7-252237, USP 5750523 and EP-A-742211.

The pyridazinone compounds (I) of the present invention and their pharmacologically acceptable salts have excellent inhitory action on vascular intimal hyperplasia in mammals such as humans, canines, bovines, equines, rabbits, mice and rats.

The pyridazinone compounds (I) of the present invention and their pharmacologically acceptable salts are administered at appropriate doses selected depending on the age, weight and conditions of the patient and usually administered to an adult human in an amount of from 0.001 mg to 5 g per day, preferably from 0.005 to 1000 mg per day, in one to several doses a day.

The pyridazinone compounds (I) of the present invention and their pharmacologically acceptable salts may be administered parenterally in the form of injections (for subcutaneous, intravenous, intramuscular or intraperitoneal injection), ointments, suppositories, aerosols, eye drops or nasal drops, orally in the form of tablets, capsules, granules, pills, powders, lozenges, chewables, syrups, solutions, emulsions or suspensions, or by using drug delivery systems such as drug-impregnated stents or other devices which allow local and sustained drug delivery.

The pyridazinone compounds (I) of the present invention and their pharmacologically acceptable salts may be formulated into various dosage forms in accordance with conventional methods commonly employed for preparation of pharmaceuticals.

For example, tablets, capsules, granules, pills, powders, lozenges or chewables for oral administration may be prepared by using an excipient (such as sugar, lactose, glucose, starch or mannitol), a binder (such as syrups, gum Arabic, gelatin, sorbitol, tragacanth, methylcellulose, or polyvinylpyrrolidone), a disintegrant (such as starch, carboxymethylcellulose or its calcium salts, microcrystalline cellulose or polyethylene glycol), a glidant (such as talc, magnesium stearate, calcium stearate or silica) or a lubricant (such as sodium laurate or glycerol) by known methods.

In the case of formulations for oral administration, organic acids such as citric acid, succinic acid, maleic acid, fumaric acid, malic acid and tartaric acid may be added to improve dissolution and absorbability.

Injections, aerosols, syrups, solutions, emulsions, suspensions, eye drops and nasal drops may be prepared by using a solvent for the active ingredient (such as water, ethyl alcohol, isopropyl alcohol, propylene glycol, 1,3-butylene glycol or polyethylene glycol), a surfactant (such as a sorbitan fatty acid ester, a polyoxyethylene sorbitan fatty acid ester, a polyoxyethylene fatty acid ester, a polyoxyethylene ether of hydrogenated castor oil or lecithin), a suspending agent (such as a cellulose derivative like the carboxymethyl sodium salt or methylcellulose or a natural rubber like tragacanth or gum Arabic) or a preservative (such as a p-hydroxybenzoate ester, benzalkonium chloride or a salt of sorbic acid) by ordinary methods. Suppositories may be prepared by using e.g., cacao butter, polyethylene glycol, lanolin, a fatty acid triglyceride or coconut oil by ordinary methods. Ointments to be absorbed percutaneously may be prepared by using e.g., white petrolatum, liquid paraffin, higher alcohols, macrogol ointment, a hydrophilic ointment or an aqueous gel base.

### EXAMPLES

Now, the present invention will be described in further detail with reference to Test Examples and Examples. However, it should be understood that the present invention is by no means restricted by these specific Examples.

In the following Test Examples and Examples, Compound A (4-bromo-6-[3-(4-chlorophenyl)propoxy]-5-(3-pyridylmethylamino)-3(2H)-pyridazinone hydrochloride) prepared by an ordinary method was used.

### Test Example 1

### Effect of Compound A on intimal hyperplasia in rat femoral arteries

### 1. Formation of intimal hypheplasia by photosensitization

Under intraperitoneal anesthesia with pentobarbital (50 mg/ mL/ kg), a catheter was placed in the left cervical vein of a Wistar rat. The left femoral artery was detached, and the probe of an ultrasonic pulse Doppler blood flowmeter was placed on the artery. The artery proximal to the site of placement of the probe was irradiated with green light (540 nm, 800,000 lx) at a distance of 5 mm from the artery. A steady blood flow was confirmed after 10 minutes of start of the irradiation, and 15 mg/mL/kg of rose bengal was injected into the left cervical vein to damage the vascular endothelium. Then, the incision was sutured.

### 2. Grouping and drug administration

Rats were divided in order as a solvent group (9 rats), a group (10 rats) to which 10 mg/kg of Compound A was administered, and a group (10 rats) to which 300 mg/kg of Cilostazol was administered. The drugs were suspended in 0.5% methylcellulose solution, adjusted so as to be administered at a dose of 5 mL/kg, and orally administered on the day of operation after confirmation of postoperative arousal, and then orally administered once a day in the morning for the subsequent three weeks.

### 3. Evaluation

Three weeks after the operation, the left femoral artery was exposed under anesthesia. The blood was washed out by physiological saline perfusion from the left ventricle at a pressure of from 75 to 90 mm Hg. The left femoral artery was perfused with phosphate buffered physiological saline (PBS) containing 1% paraformaldehyde and 2% glutaraldehyde for fixation and dissected out. For comparison, the undamaged right femoral artery was also dissected out. The dissected femoral arteries were stored in 10% neutral buffered formalin. The femoral arteries were serially sectioned to a 0.5 mm thickness to make pathological preparations and stained with hematoxylin-eosin (HE). The medial areas and the intimal area in transverse sections of the femoral arteries were measured as indices of intimal hyperplasia with a computer image analyzer, and the ratio of the medial area / the intimal area (the I/M ratio) was calculated.

### 4. Statistical Analysis

The results are expressed as average ± standard deviation. The intimal areas and the I/M ratios were analyzed for distribution uniformity in each group by Bartlett's test. If the distribution was uniform, one-way analysis of variance was done, and the averages for the solvent group and the respective drug-treated groups were analyzed by Dunnett's test to determine if there was significant difference between them. When the distribution was not uniform, the Kruskal-Wallis rank test was done, and if there was significant difference between the solvent group and a drug-treated group, the average ranks for the two groups were analyzed by Dunnett's test. The difference between two groups was considered to be significant if p < 0.05 (two-sided). Results

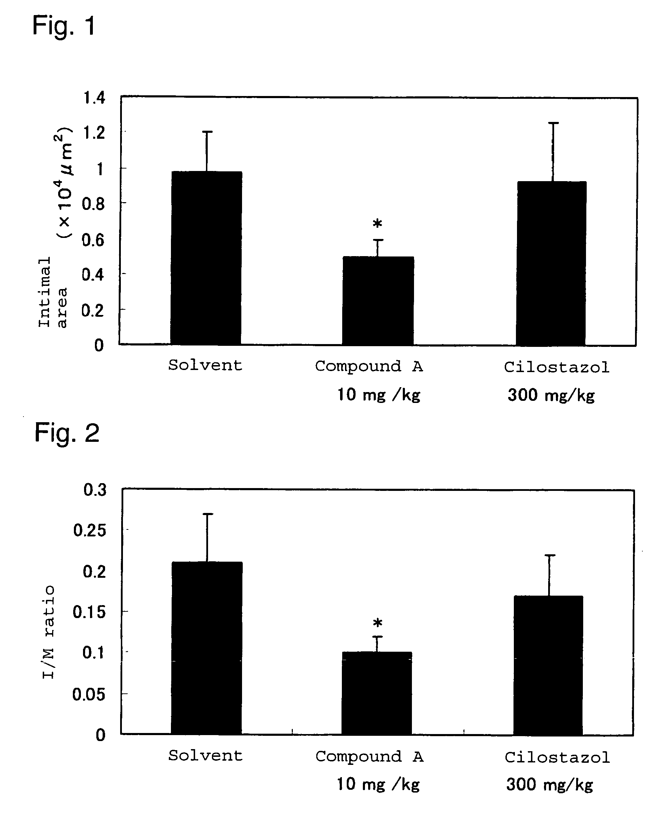

The results are shown in Fig. 1. Compound A significantly reduced the area of the tunica intima and the I/M ratio when orally administered at a dose of 10 mg/kg and turned out to have intimal hyperplasia inhibitory action. 300 mg/kg of Cilostazol showed no action.

### EXAMPLE 1 (Tablets)

10 g of Compound A, 20 g of lactose, 5 g of starch, 0.1 g of magnesium stearate and 7 g of calcium carboxymethylcellulose, 42.1 g in total, were mixed by an ordinary method and made into sugar-coated tablets each containing 50 mg of Compound A.

### EXAMPLE 2 (Tablets)

Tablets containing 10.0 mg of Compound A as the active ingredient, 5.0 mg of citric acid as an organic acid, 123.0 mg of lactose as an excipient, 4.0 mg of hydroxypropylcellulose as a binder, 7.0 mg of croscarmellose sodium as a disintegrant and 1.0 mg of magnesium stearate as a glidant were prepared.

### EXAMPLE 3 (Capsules)

10 g of Compound A, 20 g of lactose, 10 g of microcrystalline cellulose and 1 g of magnesium stearate, 41 g in total, were mixed by an ordinary method and put in gelatin capsules to obtain capsules each containing 50 mg of Compound A.

### EXAMPLE 4 (Aerosol suspension)

The following ingredients (A) were mixed, and the resulting liquid mixture was loaded into a valved vessel. The propellant (B) was injected through the valve nozzle at 20°C to a gauge pressure of about 2.46 to 2.81 mg/cm² to obtain an aerosol suspension.
(A): Compound A 0.25 mass%, isopropyl myristate 0.10 mass%, ethanol 26.40 mass%
(B): a 60-40 (mass ratio) mixture of 1,2-dichlorotetrafluoroethane and 1-chloropentafluoroethane: 73.25 mass%

### INDUSTRIAL APPLICABILITY

A novel intimal hyperplasia inhibitor containing a pyridazinone compound or a pharmacologically acceptable salt thereof as an active ingredient is provided.

The entire disclosure of Japanese Patent Application No. 2004-32551 (filed on February 9, 2004) including specification, claims, drawings and summary are incorporated herein by reference in its entirety.

## Claims

1. A vascular intimal hyperplasia inhibitor containing a 3(2H)-pyridazinone compound represented by the formula (I) or a pharmacologically acceptable salt thereof: 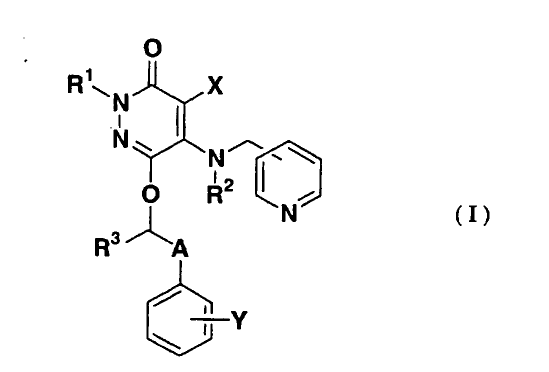 wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group.

2. The vascular intimal hyperplasia inhibitor according to Claim 1, wherein the compound represented by the formula (I) is one wherein in the formula (I), R¹ and R² are hydrogen atoms, R³ is a hydrogen atom or a C₁₋₄ alkyl group, X is a halogen atom, Y is a halogen atom or a hydrogen atom, and A is a C₁₋₅ alkylene which may be substituted with a hydroxyl group.

3. The vascular intimal hyperplasia inhibitor according to Claim 1, wherein the compound represented by the formula (I) is 4-bromo-6-[3-(4-chlorophenyl)propoxy]-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.

4. The vascular intimal hyperplasia inhibitor according to Claim 1, 2 or 3, wherein the pharmacologically acceptable salt is an organic acid salt or an inorganic acid salt.

5. The vascular intimal hyperplasia inhibitor according to Claim 1, 2 or 3, wherein the pharmacologically acceptable salt is a hydrochloride.
